# EUROPÄISCHE PATENTSCHRIFT

(11) **EP 3 490 641 B1**
(45) Veröffentlichungstag und Bekanntmachung des Hinweises auf die Patenterteilung: **25.03.2020**
(21) Anmeldenummer: 17734222.7
(22) Anmeldetag: 21.06.2017
(51) Int. Cl.: A61M 5/20, A61M 5/32, A61M 5/50

(54) **INJEKTIONSVORRICHTUNG MIT EINER ABDECKKAPPE UND EINER EINRICHTUNG ZUR VERHINDERUNG DES WIEDERAUFSETZENS DER ABDECKKAPPE**
INJECTION DEVICE COMPRISING A COVER CAP AND A SYSTEM FOR PREVENTING THE COVER CAP FROM BEING REMOUNTED
DISPOSITIF D'INJECTION DOTÉ D'UN CAPUCHON PROTECTEUR ET D'UN DISPOSITIF EMPÊCHANT LE REPOSITIONNEMENT DU CAPUCHON PROTECTEUR

(30) Priorität: 26.07.2016 CH 9592016
(43) Veröffentlichungstag der Anmeldung: 05.06.2019
(73) Patentinhaber: Tecpharma Licensing AG, 3401 Burgdorf (CH)
(72) Erfinder: BERNHARD, Mario, 3400 Burgdorf (CH); TSCHIRREN, Markus, 3400 Burgdorf (CH); TSCHING, Tashi-Panso, 4600 Olten (CH)
(86) Internationale Anmeldenummer: PCT/CH2017/000061
(87) Internationale Veröffentlichungsnummer: WO 2018/018164

(56) Entgegenhaltungen:
- EP-A1- 2 745 866
- WO-A1-2008/113199
- WO-A2-2011/047298
- US-A- 4 571 242

## Beschreibung

Die Erfindung betrifft eine Injektionsvorrichtung zur Ausschüttung oder Verabreichung eines flüssigen Produkts, insbesondere eines Medikaments. Insbesondere betrifft die Erfindung einen Mechanismus für die Injektionsvorrichtung, mit dem das Wiederaufsetzen einer Abdeckkappe, nachdem sie vom distalen Ende der Injektionsvorrichtung entfernt wurde, verhindert wird.

Der Begriff "Medikament" umfasst hier jede fließfähige medizinische Formulierung, welche geeignet ist zur kontrollierten Verabreichung durch ein Mittel wie eine Kanüle oder Hohlnadel hindurch, beispielsweise umfassend eine Flüssigkeit, eine Lösung, ein Gel oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen, sowohl in fester (suspendierter) oder flüssiger Form, aber auch Polysaccharide, Vaccine, DNS oder RNS oder Oglionukleotide, Antikörper oder Teile von Antikörpern sowie geeignete Basis-, Hilfs- und Trägerstoffe.

Aus dem Stand der Technik sind Injektionsvorrichtungen bekannt, in denen eine Spritze, die auch als Fertigspritze bezeichnet wird, angeordnet ist. Die Fertigspritze kann in einem Gehäuse der Injektionsvorrichtung verschiebbar oder unverschiebbar angeordnet sein. Es ist bekannt, an dem distalen Ende des Gehäuses eine Abdeckkappe anzubringen, die vor einer Verwendung der Injektionsvorrichtung zur Verabreichung des Produkts vom Gehäuse abgenommen oder entfernt wird. Oftmals wird die Nadel durch eine Nadelschutzkappe, die an der Spritze befestigt ist, umgeben und in Bezug auf die Umgebung steril gehalten. Aus dem Stand der Technik sind Ausführungen bekannt, in denen die Nadelschutzkappe über mindestens ein Eingriffselement mit der Abdeckkappe verbunden ist, wodurch die Nadelschutzkappe beim Entfernen der Abdeckkappe mitentfernt wird, indem die Eingriffselemente die Nadelschutzkappe mitnehmen. Gleichwohl ist es aber auch denkbar, dass zunächst die Abdeckkappe von dem Gehäuse und in einem separaten Schritt die Nadelschutzkappe von dem Produktbehälter entfernt wird.

In Ausführungen, in denen die Nadelschutzkappe zusammen mit der Abdeckkappe entfernt wird, kann bei den Injektionsvorrichtungen aus dem Stand der Technik die Abdeckkappe zusammen mit der Nadelschutzhülse wieder aufgesetzt werden, beispielsweise wenn sich der Benutzer der Injektionsvorrichtung entschieden hat, die Injektionsvorrichtung doch noch nicht verwenden zu wollen. Durch das Abnehmen der Nadelschutzhülse und das Wiederaufsetzen kann ein Benutzer irrig davon ausgehen, dass die Nadel noch steril ist, insbesondere dann, wenn längere Zeit verstrichen ist und sich der Verwender nicht mehr daran erinnert, dass er die Nadelschutzhülse schon abgenommen hatte.

Aus dem Stand der Technik sind auch Ausführungen von Injektionsvorrichtungen, insbesondere von Autoinjektoren, bekannt, die eine Auslösesicherung aufweisen, die das ungewollte Verschieben eines z. B. als Nadelschutzhülse ausgestalteten Auslöseglieds in die proximale Richtung verhindern, wenn die Abdeckkappe am Gehäuse aufgesetzt ist. Das Wiederaufsetzen der Abdeckkappe kann dazu führen, dass der Mechanismus der Auslösesicherung derart manipuliert wird, dass der Mechanismus auch dann das Verschieben des Auslöseglieds in die proximale Richtung verhindert, wenn die Abdeckkappe abgenommen ist und dadurch die Verschiebung des Auslöseglieds in die proximale Richtung eigentlich möglich sein sollte. Ein Autoinjektor mit einer Auslösesicherung wird z. B. in der EP 2 745 866 A1 beschrieben, dessen Auslöseglied ein vorzugsweise federnd angeordnetes Sperrmittel aufweist, wobei das Sperrmittel von der Abdeckkappe in eine Sperrposition, in der das Sperrmittel einem von dem Autoinjektor gebildeten Anschlag gegenüberliegt, auslenkbar ist, wobei das Sperrmittel in einer Freigabeposition, die das Sperrmittel einnimmt, wenn die Abdeckkappe abgenommen ist, an dem Anschlag vorbei bewegbar ist. Durch das Wiederaufsetzen oder unsachgemäße Wiederaufsetzen der Abdeckkappe kann es vorkommen, dass das Sperrmittel von der Abdeckkappe oder einem Abschnitt davon in die Sperrposition gedrückt und dauerhaft in dem Lagerzustand des Autoinjektors gehalten wird, wodurch - je nach Material und/oder mit zunehmender Lagerzeit - eine dauerhafte Verformung des Sperrmittels bewirkt wird, so dass sich das Sperrmittel auch bei abgenommener Abdeckkappe nicht mehr aus seiner Sperrposition bewegt, wodurch das Auslöseglied gegen die Bewegung in die Auslöseposition blockiert ist, auch wenn die Abdeckkappe vom Gehäuse abgenommen wurde.

Es ist eine Aufgabe der Erfindung, eine Injektionsvorrichtung anzugeben, welche die Gefahr von Fehlanwendungen im Zusammenhang mit dem Entfernen von einer Abdeckkappe und insbesondere Wiederanbringen der Abdeckkappe an das Gehäuse der Injektionsvorrichtung zu verringern.

Die Aufgabe wird mit der Injektionsvorrichtung nach Anspruch 1 gelöst. Vorteilhafte Weiterentwicklungen ergeben sich aus den abhängigen Ansprüchen, der Beschreibung und den Figuren.

Die Erfindung geht von einer Injektionsvorrichtung, wie z. B. von einem Autoinjektor, zur Ausschüttung eines flüssigen Produkts, insbesondere eines Medikaments, aus. Die Injektionsvorrichtung umfasst ein Gehäuse, welches vorzugsweise länglich und/oder hülsenförmig ist. Das Gehäuse kann beispielsweise dazu dienen, von dem Verwender der Vorrichtung mit einer Hand umgriffen zu werden und/oder einen Produktbehälter und/oder einen Mechanismus zum Ausschütten des Produkts aufzunehmen. In dem Gehäuse ist ein Produktbehälter angeordnet. Der Produktbehälter kann insbesondere eine Spritze sein, welche einen Spritzenkörper aufweist, an dessen distalem Ende eine Injektionsnadel fest angeordnet ist. Der vorzugsweise zylindrische Spritzenkörper umgibt einen Kolben, der in Bezug auf den Spritzenkörper verschiebbar ist und für die Produktausschüttung zu dem distalen Ende hin verschoben wird, wodurch das zwischen dem Kolben und der Injektionsnadel angeordnete flüssige Produkt, insbesondere Medikament, durch die Injektionsnadel aus dem Produktbehälter ausgeschüttet wird. Der Spritzenkörper kann an seinem proximalen, d. h. hinteren oder der Injektionsnadel entgegengesetzten Ende einen Flansch, der z. B. als Fingerflansch ausgestaltet und bezeichnet werden kann, aufweisen. Eine derartig ausgebildete Spritze ist als Standardspritze erhältlich, so dass für die Injektionsvorrichtung nicht zwingend eine speziell angepasste Spritze entwickelt werden braucht. Der Kolben liegt dichtend und verschiebbar an dem Innendurchmesser des Spritzenkörpers an.

Das vorzugsweise längliche, hülsenförmige Gehäuse bildet die Längsachse der Injektionsvorrichtung. Das Gehäuse ist vorzugsweise zylindrisch, beispielsweise kreiszylindrisch, polygonzylindrisch oder allgemein im Querschnitt unrund, wodurch ein Wegrollen der Injektionsvorrichtung im Gegensatz zu einem kreiszylindrischen Gehäuse verhindert werden kann. Der Produktbehälter ist in dem Gehäuse angeordnet. Beispielsweise kann der Behälter verschiebbar in dem Gehäuse angeordnet sein, d. h. für ein automatisches Einstechen relativ zu dem Gehäuse in die distale Richtung verschiebbar sein, so dass die Nadelspitze aus einer Öffnung am distalen Ende der Injektionsvorrichtung hervortritt und automatisch in den Patienten eingestochen werden kann. Optional kann bei einer solchen Vorrichtung die Nadelspitze nach erfolgter Produktausschüttung in das distale Ende der Vorrichtung hinein bewegt werden, insbesondere der Produktbehälter relativ zu dem Gehäuse in die proximale Richtung bewegt werden.

In Ausführungen kann der Produktbehälter entlang der Längsachse unverschiebbar in dem Gehäuse aufgenommen sein, insbesondere mittels eines Produktbehälterhalters oder Spritzenhalters, der den Produktbehälter axial fest hält und axial fest mit dem Gehäuse verbunden, insbesondere verrastet ist. Insbesondere kann die Nadelspitze distal über das distale Ende des Gehäuses stehen. Hierdurch kann die Nadel mittels einer Bewegung des Gehäuses zum Patienten hin in die Einstichstelle eingestochen werden.

Die Injektionsvorrichtung kann ein Vortriebsglied, wie z. B. eine Kolbenstange aufweisen, wobei das Vortriebsglied zur Ausschüttung des in dem Produktbehälter enthaltenen Produkts entlang der Längsachse in die distale Richtung verschiebbar ist. Beispielsweise kann das Vortriebsglied manuell, d. h. durch Muskelkraft des Verwenders in die distale Richtung verschoben werden. Alternativ kann das Vortriebsglied durch einen Energiespeicher, wie z. B. eine Feder, einen Druckspeicher oder einen Gasgenerator zur Ausschüttung des in dem Produktbehälter enthaltenen Produkts entlang der Längsachse in die distale Richtung verschoben werden. Die verschiedenen Möglichkeiten, das Vortriebsglied zur Produktausschüttung in die distale Richtung zu verschieben, sind vielfältig und an sich bekannt.

Die Injektionsvorrichtung umfasst eine Abdeckkappe, welche an dem distalen Ende des Gehäuses angebracht ist, insbesondere in dem Auslieferungszustand des Autoinjektors. Die Abdeckkappe ist von dem Gehäuse abnehmbar, insbesondere abziehbar oder abdrehbar, insbesondere abschraubbar. Zum Entfernen der Abdeckkappe führt diese in Bezug auf das Gehäuse zumindest eine Bewegungskomponente entlang der Längsachse in die distale Richtung in Bezug auf das Gehäuse aus. Zum Vorbereiten der Injektionsvorrichtung für die bestimmungsgemäße Verwendung wird die Abdeckkappe von dem Gehäuse abgenommen. In Ausführungsformen mit einem Auslöseglied, welches zur Initiierung des Einstechens und/oder des Ausschüttens des Produkts relativ zu dem Gehäuse in die proximale Richtung verschoben wird, kann die Abdeckkappe z. B. an dem Gehäuse befestigt sein und das distale Ende des Auslöseglieds umgeben, wobei es insbesondere einen Zugriff auf das distale Ende des Auslöseglieds verhindert. Wenn die Abdeckkappe von dem Gehäuse abgenommen ist, ist der Zugriff auf das Auslöseglied freigegeben, so dass es an die Einstichstelle angesetzt werden kann.

Die Abdeckkappe kann in ihrer insbesondere vollständig, auf die Injektionsvorrichtung oder dem Gehäuse angebrachten Position kraft- und/oder formschlüssig mit dem Gehäuse verbunden, wie z. B. verschnappt sein. Beispielsweise kann die Abdeckkappe ein Eingriffsglied und das Gehäuse ein Eingriffsgegenglied aufweisen, die ineinandergreifen, wenn die Abdeckkappe insbesondere vollständig an dem Gehäuse angebracht ist. Selbstverständlich ist dieser Eingriff beispielsweise durch Bewegen der Abdeckkappe lösbar, um ein Abnehmen der Abdeckkappe von dem Gehäuse zu ermöglichen.

Erfindungsgemäß weist die Injektionsvorrichtung einen Mechanismus auf, der das Wiederanbringen der Abdeckkappe an das distale Ende des Gehäuses verhindert. Unter einem Wiederanbringen wird das Anbringen verstanden, nachdem die Abdeckkappe aus ihrer insbesondere vollständig an dem distalen Ende des Gehäuses angebrachten Position von dem Gehäuse abgenommen wurde und danach wieder an dem Gehäuse befestigt wird, insbesondere vollständig angebracht wird.

In Ausführungen der Injektionsvorrichtung kann der Produktbehälter eine insbesondere unlösbar oder alternativ lösbar mit ihm verbundene Injektionsnadel aufweisen. Die Injektionsnadel kann von einer Nadelschutzkappe umgeben sein, die an dem Produktbehälter, insbesondere einem Nadelhalteabschnitt, der das proximale Ende der Nadel umgibt, insbesondere unlösbar einfasst, lösbar befestigt sein kann. Der Nadelhalteabschnitt kann sich distal an den zylindrischen Spritzenkörper anschließen. Die Nadelschutzkappe kann beispielsweise reib- oder formschlüssig oder kombiniert reib- oder formschlüssig an dem Produktbehälter, insbesondere an oder auf dem Nadelhalteabschnitt befestigt sein. Die Nadelschutzkappe umschließt die Injektionsnadel und dichtet sie in Bezug auf die Umgebung steril ab. Die Nadelschutzkappe kann z. B. als so genanntes "soft needle shield" oder "rigid needle shield" ausgestaltet sein, wie es aus dem Stand der Technik bekannt ist. Ein soft needle shield umfasst oder besteht aus einem elastomeren, beispielsweise auf Gummi- oder Kautschukbasis gebildeten, vorzugsweise kappenförmigen Teil, welches die Nadel umgibt. Das soft needle shield weist an seinem Außenumfang eine weiche, wie z. B. aus einem gummi- oder kautschukartigen Material gebildete Oberfläche auf. Ein rigid needle shield weist zumeist mehrere Teile auf, insbesondere ein elastomeres kappenförmiges inneres Teil und ein aus einem festen oder festeren, d. h. nicht elastomeren Kunststoff hergestelltes hülsenförmiges oder kappenförmiges äußeres Teil, welches das innere Teil aufnimmt und damit im Wesentlichen unlösbar verbunden ist. Das äußere hülsen- oder kappenförmige Teil umgibt das innere kappenförmige Teil und ist mit der inneren Kappe beispielsweise unlösbar verbunden, so dass die äußere und innere Kappe eine Einheit bilden. Das äußere Teil kann aus einem härteren Kunststoff gebildet sein als das innere Teil. Das äußere Teil kann beispielsweise aus Polyethylen, Polystyrol, Polypropylen oder einem anderen geeigneten Kunststoff gebildet sein. Das innere Teil kann beispielsweise aus Gummi oder Kautschuk oder einem anderen geeigneten, insbesondere elastomeren Material gebildet sein.

In Weiterbildungen kann die Abdeckkappe mit der Nadelschutzkappe derart verbunden sein, dass das Abnehmen der Abdeckkappe die Nadelschutzkappe von dem Produktbehälter entfernt. Die Abdeckkappe, welche mit einem oder mehreren Eingriffselementen gekoppelt sein kann, kann über das mindestens eine Eingriffselement so mit der Nadelschutzkappe verbunden sein, dass das Entfernen der Abdeckkappe von der Injektionsvorrichtung oder dessen Gehäuse das Entfernen der Nadelschutzkappe von dem Produktbehälter bewirkt. Beispielsweise kann zumindest ein Teil der Bewegung oder die gesamte Bewegung der Abdeckkappe in die distale Richtung auf das Eingriffselement übertragen werden, d. h., dass das Eingriffselement von der Abdeckkappe mitgenommen wird, so dass das Eingriffselement die Nadelschutzkappe von dem Produktbehälter, insbesondere dem Nadelhalteabschnitt, abzieht.

Das mindestens eine Eingriffselement kann beispielsweise von der Abdeckkappe gebildet sein oder von einem beispielsweise hülsenförmigen Entfernerelement gebildet sein, welches an der Abdeckkappe entlang der Längsachse unverschiebbar oder begrenzt verschiebbar angeordnet ist, so dass das Entfernerelement beim Entfernen der Abdeckkappe von der Abdeckkappe mitgenommen wird, insbesondere entlang der Längsachse L.

Das mindestens eine Eingriffselement kann an die Nadelschutzkappe angreifen oder in die Nadelschutzkappe eingreifen, so dass die Nadelschutzkappe beim Abziehen der Abdeckkappe von dem Eingriffselement von dem Produktbehälter entfernt wird. Beispielsweise kann sich der zylindrische Abschnitt des Produktbehälters an seinem distalen Ende verjüngen und z. B. in den Nadelhalteabschnitt übergehen. Zwischen diesem sich verjüngenden Ende und dem proximalen Ende der Nadelschutzkappe kann beispielsweise ein Spalt gebildet sein, in dem das Eingriffselement angeordnet ist. Alternativ kann das Eingriffselement in die Außenumfangsfläche der Nadelschutzkappe eingreifen. Das Eingriffselement kann im Auslieferungszustand bereits an oder in den Produktbehälter an- oder eingreifen oder in den Eingriff erst während des und durch das Abnehmen der Abdeckkappe von dem Gehäuse gebracht werden.

Die Abdeckkappe und die Nadelschutzkappe können während und/oder nach dem Abnehmen der Abdeckkappe von dem Gehäuse eine Einheit derart bilden, dass die Nadelschutzkappe in der Abdeckkappe aufgenommen ist und nicht oder nicht ohne weiteres von der Abdeckkappe lösbar ist. Die Abdeckkappe und die Nadelschutzkappe bilden somit eine handhabbare Einheit.

Da beim Entfernen der Abdeckkappe von dem Gehäuse in diesen Ausführungen die Nadelschutzkappe von der Injektionsnadel abgezogen wird, kann die Injektionsnadel in den Kontakt mit der Umgebungsatmosphäre kommen, wodurch sie nicht mehr als steril gilt. Ein Wiederaufsetzen oder Wiederanbringen der Abdeckkappe auf oder an das Gehäuse könnte den Anschein verleihen, dass die Nadel noch steril ist. Durch den erfindungsgemäßen Mechanismus, der das Wiederanbringen der Abdeckkappe an das distale Ende des Gehäuses verhindert, insbesondere das vollständige Wiederanbringen, z. B. so dass die Nadelschutzhülse, insbesondere deren Eingriffsglied mit dem Gehäuse, insbesondere dem Eingriffsgegenglied des Gehäuses, verschnappt, wird verhindert, dass beim Benutzer durch ein vollständiges Wiederanbringen der Anschein erweckt wird, die Nadel sei noch steril. Somit wird die Gefahr einer fehlerhaften Anwendung der Injektionsvorrichtung verringert.

Alternativ oder zusätzlich zu den vorher genannten Ausführungsformen kann die Injektionsvorrichtung ein insbesondere hülsenförmiges Auslöseglied aufweisen, welches in seiner Ausgangsposition über das distale Ende des Gehäuses ragt und für die Auslösung der Ausschüttung relativ zu dem Gehäuse aus der Ausgangsposition in eine proximale Richtung in eine Auslöseposition bewegbar ist, wenn die Abdeckkappe von dem Gehäuse abgenommen ist, und gegen die Bewegung in die Auslöseposition blockiert ist, wenn die Abdeckkappe an dem Gehäuse angeordnet ist. Unter einer Blockierung wird insbesondere nicht das bloße Verhindern des Zugriffs auf das hülsenförmige Auslöseglied verstanden, sondern vielmehr eine Blockierung dergestalt, dass zwei Teile aneinander anstoßen. Das Auslöseglied dient primär zur Auslösung der Produktausschüttung oder gegebenenfalls einer optionalen Einstechsequenz. Optional kann das Auslöseglied als Nadelschutzhülse dienen und als solche bezeichnet oder ausgestaltet werden. Das Auslöseglied, welches bei abgenommener Abdeckkappe das distale Ende der Injektionsvorrichtung bildet, kann eine Öffnung für die Injektionsnadel aufweisen, wobei die Injektionsnadel durch die Öffnung hindurchtreten kann. Das Auslöseglied kann in seiner Ausgangsposition in Bezug auf die Nadelspitze so angeordnet sein, dass das Auslöseglied, insbesondere die Nadelschutzhülse, distal über die Nadelspitze steht oder dass die Nadelspitze distal über das distale Ende des Auslöseglieds steht. Das Auslöseglied ist relativ zu dem Gehäuse aus seiner Ausgangsposition in die proximale Richtung um einen Betätigungshub in eine betätigte Position, insbesondere Betätigungsposition, verschiebbar, insbesondere in das Gehäuse verschiebbar, so dass die Nadel aus dem distalen Ende bzw. durch die Öffnung des Auslöseglieds hervortritt oder weiter hervortritt. Beispielsweise kann das Auslöseglied um einen Nadelschutzhub aus der Auslöseposition relativ zu dem Gehäuse in die distale Richtung in eine Nadelschutzposition verschoben werden, in der das distale Ende des Auslöseglieds, insbesondere der Nadelschutzhülse, distal über die Nadelspitze steht, um nach erfolgter Verwendung der Vorrichtung bzw. nach erfolgter Produktausschüttung eine Verletzungsgefahr, die von einer frei liegenden Nadelspitze ausgehen würde, zu verringern.

Das Auslöseglied kann z. B. gegen die Kraft einer Feder, die z. B. als Rückstellfeder oder Nadelschutzfeder bezeichnet werden kann, in die proximale Richtung verschoben werden, wobei die Feder das Auslöseglied aus der Position, in der ein Sperrmittel die Bewegung des Auslöseglieds in die proximale Richtung sperrt, in die distale Richtung verschieben kann oder aus der Auslöseposition in die distale Richtung, z. B. bis in die Nadelschutzposition verschieben kann.

Das insbesondere hülsenförmige Auslöseglied, welches in seiner Ausgangsposition über das distale Ende des Gehäuses ragt, ist für die Auslösung der Ausschüttung relativ zu dem Gehäuse aus der Ausgangsposition in die proximale Richtung in die Auslöseposition bewegbar, wenn die Abdeckkappe von dem Gehäuse abgenommen ist. Das Auslöseglied ist mit einem Antriebsmechanismus, der vorzugsweise von dem Gehäuse umgeben wird, derart gekoppelt, dass der Antriebsmechanismus die Produktausschüttung oder gegebenenfalls eine Einstechsequenz freigibt, wenn das Auslöseglied in seiner Auslöseposition ist oder die Auslöseposition erreicht.

In Weiterbildungen kann das Auslöseglied gegen die Bewegung in die Auslöseposition blockiert werden, wenn die Abdeckkappe am Gehäuse angeordnet ist. Beispielsweise kann das Auslöseglied gar nicht oder nur um einen Hub in die proximale Richtung bewegt werden, der geringer ist als der Auslösehub, d. h. der Hub, den das Auslöseglied bei der Bewegung aus der Ausgangsposition in die Auslöseposition ausführt.

Besonders vorteilhaft lässt sich die vorliegende Erfindung bei dem Autoinjektor aus der EP 2 745 866 A1 einsetzen, wobei der Inhalt dieser Druckschrift durch Bezugnahme in die vorliegende Anmeldung aufgenommen wird.

Das Auslöseglied wird gegen die Bewegung in die Auslöseposition blockiert, wenn die Abdeckkappe am Gehäuse angeordnet ist. Beispielsweise kann das Auslöseglied gar nicht oder nur um einen Hub in die proximale Richtung bewegt werden, der geringer ist als der Auslösehub, d. h. der Hub, den das Auslöseglied bei der Bewegung aus der Ausgangsposition in die Auslöseposition ausführt.

Beispielsweise kann die Injektionsvorrichtung ein Vortriebsglied, das zumindest während der Produktausschüttung auf den Kolben wirkt, insbesondere an dem Kolben anliegt, und eine Ausschüttfeder, die auf das Vortriebsglied wirkt, wie zum Beispiel sich insbesondere mit ihrem distalen Ende an dem Vortriebsglied abstützt, aufweisen. Das Vortriebsglied kann zum Bespiel hülsenförmig sein und eine Schulter, die zum Beispiel im Bereich des distalen Endes des Vortriebsglieds angeordnet ist, bilden, an der sich das distale Ende der Ausschüttfeder abstützen kann. Die Ausschüttfeder kann vorzugsweise innerhalb des z. B hülsenförmigen Vortriebsglieds angeordnet sein. Die Ausschüttfeder ist vorzugsweise eine als Druckfeder wirkende Wendelfeder, die zum Bespiel aus Metall gebildet ist. Die Ausschüttfeder kann so stark vorgespannt sein, insbesondere im Auslieferungszustand des Autoinjektors, dass sie bzw. die in ihr gespeicherte Energie ausreicht, um das Produkt aus dem Produktbehälter durch Verschieben des Vortriebsglieds um einen Ausschütthub im Wesentlichen vorständig auszuschütten. Durch die Verschiebung des Vortriebsglieds um den Ausschütthub wird auch der Kolben verschoben. Sofern im Auslieferungszustand zwischen dem Kolben und dem Vortriebsglied ein Abstand besteht, ist der Ausschütthub des Kolbens kleiner als der Ausschütthub des Vortriebsglieds, was bevorzugt ist, da der Kolben bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand und nicht erst bei der Produktausschüttung an dem Kolben anliegt. Sofern im Auslieferungszustand das Vortriebsglied bereites an dem Kolben anliegt, entspricht der Ausschütthub des Kolbens dem Ausschütthub des Vortriebsglieds. Das proximale Ende der Ausschüttfeder kann sich an dem Gehäuse oder einem gehäusefesten Element, insbesondere auch einen nur zeitweise zu dem Gehäuse axialfesten Element abstützen.

In Weiterbildungen kann das Auslöseglied ein z. B. gelenkig oder vorzugsweise federnd angeordnetes Sperrmittel aufweisen oder bilden. Das Auslöseglied kann einen hülsenförmigen Hauptkörper aufweisen, an dem das Sperrmittel gelenkig oder federnd angeordnet, insbesondere einstückig mit dem Hauptkörper gebildet ist. Das Sperrmittel ist von der Abdeckkappe in eine Sperrposition, in der das Sperrmittel einem von der Injektionsvorrichtung gebildeten Anschlag gegenüberliegt, insbesondere in einer Flucht mit dem Anschlag ist, auslenkbar. Das Sperrmittel ist in seiner Freigabeposition, die das Sperrmittel einnimmt, wenn die Abdeckkappe abgenommen ist, an dem Anschlag vorbei bewegbar, d. h. nicht in der Flucht mit dem Anschlag. Die Abdeckkappe kann somit einen Aktuator bilden, der das Sperrmittel in die Freigabeposition drückt oder bewegen kann, insbesondere relativ zu dem Hauptkörper, wenn die Abdeckkappe an dem Gehäuse angeordnet ist, insbesondere vollständig an dem Gehäuse angeordnet ist. Wenn die Abdeckkappe von dem Gehäuse abgenommen ist, fehlt dieser Aktuator, wodurch das Sperrmittel nicht mehr in seine Sperrposition gedrückt wird oder werden kann. Vorteilhaft wird dadurch erreicht, dass der Benutzer des Autoinjektors mit dem Entfernen der Abdeckkappe von dem Gehäuse gleichzeitig das Auslöseglied für die Bewegung aus der Ausgangsposition in die Auslöseposition entsperrt. Wenn die Abdeckkappe an dem Gehäuse angeordnet ist, ist das Auslöseglied gegen die Bewegungen der Auslöseposition blockiert oder gesperrt. Durch das Sperrmittel kann der Autoinjektor nicht mehr durch die Massenträgheit des Auslöseglieds bei starker Erschütterung ausgelöst werden.

Insbesondere kann eine Aktuatorfläche, welche das Sperrmittel in die Sperrposition drückt, in Bezug auf das Sperrmittel so angeordnet sein, dass die Abdeckkappe, insbesondere die Aktuatorfläche und/oder die Haltefläche, das Sperrmittel erst dann in seine Sperrposition auslenkt, wenn das Auslöseglied aus seiner Ausgangsposition in die proximale Richtung bewegt wird, hierdurch wird sichergestellt, dass sich das Sperrmittel nur dann in die Sperrposition bewegt, wenn bei aufgesetzter Abdeckkappe das Auslöseglied aus seiner Ausgangsposition in die proximale Richtung bewegt wird, wie z. B. bei einer Erschütterung, die z. B. durch Herunterfallen der Injektionsvorrichtung auf den Boden verursacht werden könnte.

Bei einer solchen Ausführungsform kann unter Umständen der Effekt auftreten, dass beim Wiederaufsetzen der Abdeckkappe das Sperrmittel in seine Sperrposition ausgelenkt wird und von der Abdeckkappe, beispielsweise deren Haltefläche, in der Sperrposition gehalten wird. Dadurch kann in Abhängigkeit von dem Material des Sperrmittels und der Zeitdauer des Auslenkens eine dauerhafte Verformung des Sperrmittels in seine Sperrposition die Folge sein, wodurch sich das Sperrmittel nicht mehr aus der Sperrposition bewegt, wenn die Kappe abgenommen wird. Durch den erfindungsgemäßen Mechanismus, der das Wiederanbringen der Abdeckkappe an das distale Ende des Gehäuses verhindert, wird somit auch verhindert, dass das Sperrmittel auch ungewollt in seine Sperrposition gebracht wird, wodurch mit dieser Maßnahme diese Fehlerquelle behoben wird.

In Weiterbildungen kann der Mechanismus oder die Injektionsvorrichtung einen Axialanschlag und einen Axialgegenanschlag aufweisen, wobei wenigstens einer aus Axialanschlag und Axialgegenanschlag durch das Abnehmen der Abdeckkappe in eine Position gebracht wird, in der das Wiederanbringen der Abdeckkappe an das distale Ende des Gehäuses dadurch verhindert wird, dass der Axialanschlag und der Axialgegenanschlag aneinander anstoßen. Das heißt, dass der Axialanschlag und der Axialgegenanschlag aneinander anstoßen, bevor die Abdeckkappe auf das Gehäuse oder vollständig auf das Gehäuse aufgesetzt ist, wenn der Versuch unternommen wird, die Abdeckkappe wieder an das Gehäuse anzubringen. Insbesondere kann wenigstens einer aus Axialanschlag und Axialgegenanschlag während des Abnehmens verformt, insbesondere elastisch verformt werden und dadurch in eine Position gebracht werden, in der das Wiederanbringen der Abdeckkappe an das distale Ende des Gehäuses verhindert wird. Unter einem Axialanschlag bzw. Axialgegenanschlag werden Strukturen, wie z.B. Flächen, verstanden, welche eine Anschlagfläche aufweist, die in Richtung der Längsachse wirken.

Der Axialanschlag kann von dem Gehäuse oder einem sich an dem Gehäuse zumindest in die proximale Richtung abstützenden Element gebildet sein. Das sich zumindest in die proximale Richtung abstützende Element kann entlang der Längslachse L axialfest oder begrenzt axialbewegbar sein, wobei zumindest die Bewegung in die proximale Richtung in Bezug auf das Gehäuse blockiert oder blockierbar ist, so dass eine auf den Axialanschlag wirkende Kraft über das Element in das Gehäuse geleitet werden kann.

Der Axialgegenanschlag kann von der Abdeckkappe oder ein sich an der Abdeckkappe zumindest in die distale Richtung abstützende Elemente gebildet sein. Das sich an der Abdeckkappe zumindest in die distale Richtung abstützende Element kann entlang der Längsachse axialfest mit der Abdeckkappe verbunden, insbesondere verschnappt oder formschlüssig verbunden sein oder begrenzt in Bezug auf die Abdeckkappe bewegbar sein, wobei bevorzugt ist, dass sich das Element zumindest in die distale Richtung an der Abdeckkappe abstützen kann, wodurch eine auf den Axialanschlag ausgeübte Kraft über das Element die Abdeckkappe geleitet wird.

Beispielsweise kann die Abdeckkappe einen Abdeckkappengrundkörper, der z. B. mit dem Gehäuse kraft- und/oder formschlüssig verbunden ist, wenn die Abdeckkappe vollständig an dem Gehäuse angebracht ist, insbesondere wenn das Eingriffsglied des Abdeckkappengrundkörpers und das Eingriffsgegenglied des Gehäuses ineinandergreifen. Die Abdeckkappe kann den Abdeckkappengrundkörper und eine Außenhülse aufweisen, die mit dem Abdeckkappengrundkörper zumindest axialfest, vorzugsweise dreh- und axialfest verbunden, insbesondere verschnappt, ist und den Axialgegenanschlag bildet. Die Außenhülse kann z. B. auf die dem Abdeckkappengrundkörper entsprechende Abdeckkappe des Autoinjektors aus der EP 2 745 866 A1 als Nachrüstelement aufgeschoben werden. Hierzu kann die Außenhülse z. B. über das distale oder proximale Ende der Injektionsvorrichtung über die Abdeckkappe bzw. den Abdeckkappengrundkörper geschoben werden, wodurch die Außenhülse mit dem Abdeckkappengrundkörper verrastet oder verschnappt.

In Weiterbildungen kann der Axialanschlag federnd, d.h. elastisch nachgiebig, angeordnet, insbesondere von oder an dem Gehäuse gebildet sein und beim oder während des Abnehmens der Abdeckkappe vom Gehäuse aus einer ausgelenkten Position in eine Sperrposition federn. Der Axialanschlag verhindert in der Sperrposition das Wiederanbringen der Abdeckkappe an das distale Ende des Gehäuses dadurch, dass der Axialanschlag und der Axialgegenanschlag aneinander anstoßen.

Alternativ oder zusätzlich kann der Axialgegenanschlag federnd, insbesondere an der Abdeckkappe oder der mit dem Abdeckkappengrundkörper verbundenen Außenhülse, angeordnet sein und beim oder während des Abnehmens der Abdeckkappe vom Gehäuse aus einer ausgelenkten Position in eine Sperrposition federn. Der Axialgegenanschlag verhindert in seiner Sperrposition das Wiederanbringen der Abdeckkappe an das distale Ende des Gehäuses dadurch, dass der Axialanschlag und der Axialgegenanschlag aneinander anstoßen.

Insbesondere können der Axialanschlag und der Axialgegenanschlag beim Versuch, die Abdeckkappe an dem Gehäuse wieder anzubringen, in einer axialen Flucht, d. h. in einer Flucht entlang der Längsachse sein, wodurch sie aneinander anstoßen.

Beispielsweise könnte die Abdeckkappe in einer oder in mehreren diskreten Drehpositionen in Bezug auf das Gehäuse aufsetzbar sein, wenn der erfindungsgemäße Mechanismus nicht vorhanden wäre, wobei erfindungsgemäß in allen der mindestens einen oder mehreren, wie zum Beispiel maximal vier verschiedenen Drehpositionen einer der genannten Axialgegenanschläge und einer der genannten Axialanschläge entlang der Längsachse in einer Flucht angeordnet sind, wodurch das Wiederanbringen der Abdeckkappe verhindert wird.

Beispielsweise kann die Anordnung des Axialanschlags oder des Axialgegenanschlags so ausgestaltet sein, dass der Axialanschlag oder der Axialgegenanschlag aus der ausgelenkten Position quer, insbesondere im Wesentlichen radial zur Längsachse, oder in Umfangsrichtung um die Längsachse in die Sperrposition federt. Beispielsweise kann der Axialanschlag und/oder der Axialgegenanschlag jeweils über einen flexiblen Arm federnd angeordnet sein, insbesondere mit dem Gehäuse einem sich an dem Gehäuse zumindest in die proximale Richtung abstützende Element bzw. an der Abdeckkappe oder einem sich an der Abdeckkappe zumindest in die distale Richtung abstützende Element angeordnet sein.

Beispielsweise kann das Gehäuse eine Getriebefläche aufweisen, welche den Axialanschlag während des Abnehmens der Abdeckkappe von dem Gehäuse aus einer Ausgangsposition in seine ausgelenkte Position federnd auslenkt. Die Getriebefläche kann z. B. so angeordnet sein, dass der Axialgegenanschlag - wie oben beschrieben - etwa radial zur Längsachse oder in Umfangsrichtung um die Längsachse ausgelenkt wird, insbesondere federnd ausgelegt wird. Aus dieser ausgelenkten Position kann der Axialgegenanschlag dann - wie oben beschrieben - in die Sperrposition federn. Der Axialgegenanschlag oder eine Abragung, welche den Axialgegenanschlag bildet, kann während des Abnehmens der Abdeckkappe an der Getriebefläche entlanggleiten. Die Getriebefläche kann beispielsweise in Bezug auf die Längsachse geneigt angeordnet sein, so dass der Axialgegenanschlag bzw. die Abragung aus der Ausgangsposition in seine ausgelenkte Position federnd ausgelenkt werden kann.

Beispielsweise kann das Gehäuse an seinem Außenumfang eine Ausnehmung aufweisen, in die eine bzw. die Abragung, die den Axialgegenanschlag bildet, in ihrer Ausgangsposition eingreift, wobei das distale Ende der Ausnehmung die Getriebefläche bilden kann. Die Ausnehmung kann z. B. ein Durchbruch durch das Gehäuse sein und gleichzeitig als Fenster zur Überwachung des Produktbehälters dienen, Wenn der Axialgegenanschlag federnd angelenkt ist, wie z. B. über den Arm, kann die federnde Anlenkung oder der Arm entspannt sein, wenn der Axialgegenanschlag oder die den Axialgegenanschlag bildende Abragung in ihrer Ausgangsposition ist oder in die Ausnehmung eingreift. Der Vorteil hierbei ist, dass die Anlenkung oder der Arm erst während des Abnehmens der Abdeckkappe von dem Gehäuse gespannt wird, so dass sichergestellt wird, dass der Axialgegenanschlag in seine Sperrposition federt.

In allgemein bevorzugten Ausführungen kann der Axialgegenanschlag proximal des Axialanschlags angeordnet sein, wenn die Abdeckkappe an dem Gehäuse angeordnet ist, insbesondere vollständig an dem Gehäuse angeordnet ist. Durch Abnehmen der Abdeckkappe kann der Axialgegenanschlag an dem Axialanschlag entlang der Längsachse vorbeibewegt werden. Wenn die Abdeckkappe vom Gehäuse entfernt ist, kann sich der Axialgegenanschlag z. B. distal des Axialanschlags befinden.

In einer alternativen Ausführung kann die Injektionsvorrichtung eine Drehhülse aufweisen, welche insbesondere an und vorzugsweise relativ zu dem Gehäuse um die Längsachse der Injektionsvorrichtung drehbar gelagert ist und an welcher der Axialanschlag gebildet ist. Insbesondere kann das Gehäuse die Drehhülse umgeben. Die Drehhülse ist vorzugsweise axial fest, kann aber auch begrenzt entlang der Längsachse verschiebbar an dem Gehäuse gelagert sein. Es ist bevorzugt, dass sich die Drehhülse ist in die proximale Richtung axialfest an dem Gehäuse abstützen kann. Die Abdeckkappe oder ein Teil oder Abschnitt der Abdeckkappe kann während des Abnehmens der Abdeckkappe von dem Gehäuse an der Drehhülse abgleiten, und die Drehhülse um die Längsachse drehen, wodurch der Axialanschlag der an der Drehhülse gebildet ist, entsprechend um die Längsachse mit der Drehhülse mitgedreht wird, d. h. in eine Sperrposition gedreht wird. Der Axialanschlag verhindert in der Sperrposition das Wiederanbringen der Abdeckkappe an das distale Ende des Gehäuses dadurch, dass der Axialanschlag und der Axialgegenanschlag aneinander anstoßen.

Die Erfindung wurde anhand von mehreren bevorzugten Ausführungen und Beispielen beschrieben. Im Folgenden wird eine besonders bevorzugte Ausführung anhand von Figuren beschrieben. Dabei offenbarten Merkmale bilden die Erfindung einzeln und in jeglicher Merkmalskombination beispielsweise auch mit einem oder mehreren Merkmalen der vorhergehenden Beschreibung vorteilhaft weiter. Es zeigen:
- Figuren 1a und 1b: eine Injektionsvorrichtung im vollständig montierten Zustand, wobei die Figur 1a eine Schnittansicht entlang der Linie A-A aus Figur 1b ist,
- Figuren 2a und 2b: die Injektionsvorrichtung aus den Figuren 1a und 1b, wobei eine Außenhülse in einer Position gezeigt wird, bevor sie mit einem Abdeckkappengrundkörper einer Abdeckkappe der Injektionsvorrichtung verbunden wird, wobei Figur 2a eine Schnittansicht entlang der Linie D-D aus Figur 2b ist,
- Figur 3: eine Schnittansicht einschließlich einer Detailansicht der Injektionsvorrichtung aus den Figuren 1a und 1b während des Entfernens der Abdeckkappe von einem Gehäuse der Injektionsvorrichtung,
- Figur 4: einen Querschnitt durch das Gehäuse der Injektionsvorrichtung,
- Figur 5: eine Schnittansicht der Injektionsvorrichtung aus den Figuren 1a und 1b, wobei die Abdeckkappe von dem Gehäuse entfernt ist, und
- Figur 6: eine Schnittansicht der Injektionsvorrichtung aus Figur 5, während des Versuchs die Abdeckkappe an dem Gehäuse wiederanzubringen.

Die Injektionsvorrichtung weist ein hülsenförmiges, längliches Gehäuse 10 mit einer Längsachse L auf. Die den Figuren dargestellte Injektionsvorrichtung entspricht dem Autoinjektor aus der EP 2 745 866 A1, aber zusätzlich mit dem erfindungsgemäßen Mechanismus, der das Wiederanbringen der Abdeckkappe 30 verhindert. Der im distalen oder hinteren Teil untergebrachte Antriebsmechanismus ist dem zuständigen Fachmann geläufig und kann zum Beispiel so ausgestaltet sein, wie in der EP 2 745 866 A1 oder der WO 2008/113199 A1 angegeben, ist aber nicht darauf beschränkt. Vielmehr kann der Mechanismus auch in Injektionsvorrichtungen Anwendung finden, bei denen eine Nadelschutzkappe 24 mit der Abdeckkappe 30 entfernt wird und beim Wiederanbringen der Abdeckkappe 30 ebenfalls wieder mit angebracht werden würde

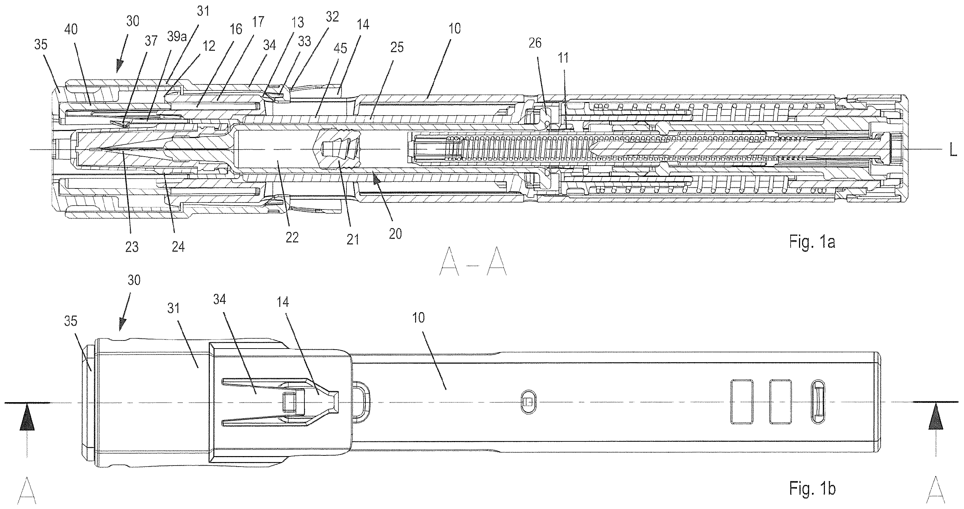

An dem distalen Ende der Injektionsvorrichtung ist in ihrem Auslieferungszustand (Figuren 1a, 1b) eine Abdeckkappe 30 angeordnet, die an dem Gehäuse 10 zum Beispiel kraft- und/oder formschlüssig befestigt ist und die vor der Verwendung des Autoinjektors abgezogen oder abgedreht, und entfernt wird.

In dem Gehäuse 10 ist ein Produktbehälter 20 in der Gestalt einer Spritze, wie zum Beispiel in Bezug auf das Gehäuse 10 entlang der Längsachse L verschiebbar oder unverschiebbar, aufgenommen. Der Produktbehälter 20 weist einen hülsenförmigen Produktbehälterabschnitt 25 auf, der im Folgenden als Spritzenkörper bezeichnet wird, der einen Kolben 21, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 23 auf, deren distales Ende von der Nadelspitze gebildet wird. Zwischen der Injektionsnadel 23 und dem Kolben 21 ist ein flüssiges Produkt, insbesondere Medikament, innerhalb des Spritzenkörpers angeordnet, wobei das flüssige Produkt durch Verschieben des Kolbens 21 in eine Ausschüttrichtung, d. h. in die distale Richtung oder zu der Injektionsnadel 23 hin, durch die hohle Injektionsnadel 23 aus dem Produktbehälter 20 ausgeschüttet wird. Der Spritzenkörper weist an seinem proximalen Ende einen sogenannten Fingerflansch 26 auf, der radial nach außen über den Außenumfang des zylindrischen Spritzenkörpers ragt.

Der Produktbehälter 20 ist in einem Produktbehälterhalter 45, der als Spritzenhalter bezeichnet wird, so aufgenommen, dass er zumindest gegen eine Bewegung entlang der Längsachse L in distale Richtung relativ zu dem Spritzenhalter gesichert ist. Der Spritzenhalter kann mit dem Gehäuse 10 formschlüssig verbunden, insbesondere verrastet sein, oder relativ zu dem Gehäuse 10 entlang der Längsachse L verschiebbar sein, je nach Ausführung der Injektionsvorrichtung. Sofern der Spritzenhalter mit dem Gehäuse 10 verrastet wird, kann das Gehäuse 10 hierfür Ausnehmungen aufweisen, in die Rastelemente, die am Spritzenhalter gebildet werden, eingreifen. Der Spritzenhalter weist mindestens eine nach innen ragende Schulter auf, an der sich ein verjüngender Abschnitt des Produktbehälters 20, der distal des zylindrischen Spritzenkörpers angeordnet ist, abstützt.

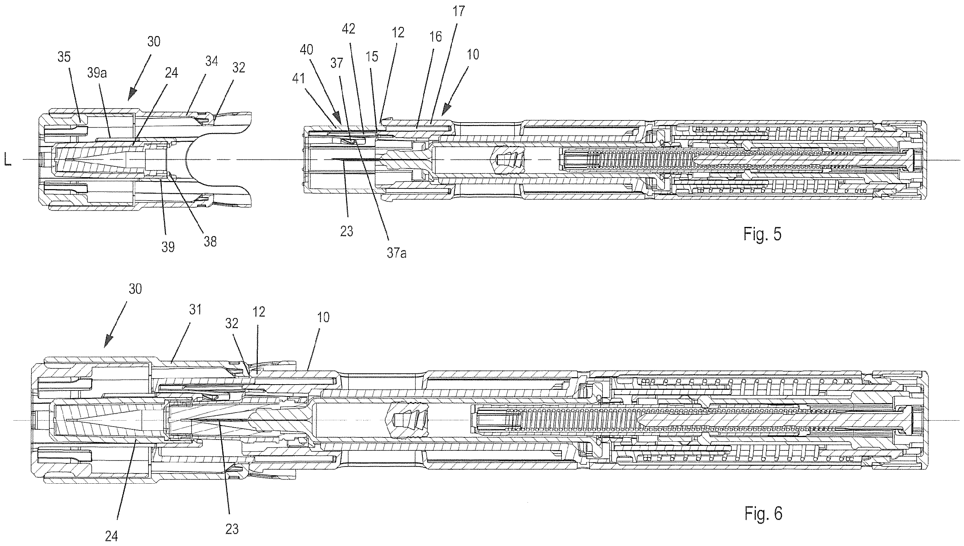

Die Injektionsvorrichtung weist an ihrem distalen Ende ein hülsenförmiges Auslöseglied 40, insbesondere Nadelschutzhülse, auf, welches in seiner Ausgangsposition (z. B. Figur 5) über das distale Ende des Gehäuses 10 ragt. Das Auslöseglied 40 wird bei aufgesetzter Abdeckkappe 30 vollständig von dieser umgeben. Das Auslöseglied 40 weist ein federnd angeordnetes Sperrmittel 37, welches zungen- oder armförmig ausgestaltet ist, auf. Das längliche Sperrmittel 37 erstreckt sich in etwa parallel zu der Längsachse L und weist mit seinem freien Ende in die proximale Richtung. Mit seinem anderen Ende ist es über einen federnden Abschnitt mit dem Auslöseglied 40, insbesondere einem hülsenförmigen Hauptkörper des Auslöseglieds 40, einstückig verbunden, insbesondere mit seinem distalen Ende. Das freie Ende des Sperrmittels 37 bildet eine Kontaktfläche 37a für einen Anschlag 15, der von dem Gehäuse 10 gebildet wird. Die Kontaktfläche 37a weist in die proximale Richtung, wobei die Anschlagfläche 15 in die distale Richtung weist. Das Sperrmittel 37 weist eine Nocke auf, die zu der Längsachse L hin abragt. Die Nocke ist zwischen dem freien Ende und dem federnden Abschnitt des Sperrmittels 37 angeordnet. Das Auslöseglied 40 weist einen hülsenförmigen Abschnitt 41 auf, zwischen dem und dem Sperrmittel 37 ein erster Spalt 42 gebildet ist. Das Gehäuse 10 weist z. B. einen hülsenförmigen Abschnitt 17 auf, wobei zwischen dem z. B. hülsenförmigen Abschnitt 17 des Gehäuses 10 und dem Anschlag 15, insbesondere einem Gehäuseabschnitt, welcher den Anschlag 15 bildet, ein zweiter Spalt 16 gebildet ist.

Das Auslöseglied 40 ist relativ zu dem Gehäuse 10 in die proximale Richtung verschiebbar, wobei der hülsenförmige Abschnitt 41 des Auslöseglieds 40 in den zweiten Spalt 16 und der Anschlag 15, insbesondere der Gehäuseabschnitt, in den ersten Spalt 42 verschoben werden oder verschiebbar sind.

Durch Verschieben des Auslöseglieds 40 in distale Richtung bis in eine Auslöseposition kann die Produktausschüttung oder ein Einstechvorgang in bekannter Weise ausgelöst werden.

In Figur 1 befindet sich die Abdeckkappe 30 in ihrer an dem Gehäuse 10 befestigten Position. Die Abdeckkappe 30 bildet eine Haltefläche 39a, die proximal der Nocke angeordnet ist, wenn sich das Auslöseglied 40 in seiner Ausgangsposition befindet (Figur 1). Distal der Haltefläche 39a weist die Abdeckkappe 30 eine Ausnehmung für die Nocke auf, so dass sich das Sperrmittel 37 durch seine federnde Anordnung in einer Freigabeposition oder Position außerhalb seiner Sperrposition befindet, wobei die Nocke in dieser Ausnehmung angeordnet ist. Die Kontaktfläche 37a ist hierbei außerhalb einer axialen Flucht entlang der Längsachse L mit dem Anschlag 15.

Wenn die Injektionsvorrichtung zum Beispiel versehentlich auf den Boden fällt, kann unter Umständen das Auslöseglied 40 aufgrund seiner Massenträgheit relativ zu dem Gehäuse 10 in die proximale Richtung verschoben werden. Um zu verhindern, dass das Auslöseglied 40 dabei bis in seine Auslöseposition verschoben wird, was zur Folge hätte, dass die Produktausschüttung ungewollt ausgelöst wird, wird die Bewegung des Auslöseglieds 40 bis in seine Auslöseposition verhindert, indem das Sperrmittel 37, insbesondere die Kontaktfläche 37a an dem Anschlag 15 anschlägt.

Wenn bei aufgesetzter Abdeckkappe 30 das Auslöseglied 40 aus seiner Ausgangsposition (Figur 1) relativ zu dem Gehäuse 10 in die proximale Richtung bewegt wird, wird das Sperrmittel 37 aus seiner Freigabeposition (Figur 1) in die Sperrposition ausgelenkt. Die Abdeckkappe 30 weist hierzu eine Aktuator- oder Getriebefläche auf, an welcher die Nocke, insbesondere eine abgeschrägte Getriebefläche der Nocke, abgleitet. Hierdurch werden die Nocke und somit auch das Sperrmittel 37 federnd ausgelenkt, insbesondere von der Längsachse L weg. Die von der Längsachse L weg weisende, d. h. nach außen weisende Haltefläche 39a, an der die Nocke dann anliegt, hält das Sperrmittel 37 in seiner Sperrposition. In der Sperrposition befindet sich die Kontaktfläche 37a in einer axialen Flucht entlang der Längsachse L mit dem Anschlag 15. Hierdurch wird bewirkt, dass die Kontaktfläche 37a an dem Anschlag 15 anschlägt und somit eine Bewegung des Auslöseglieds 3 bis in seine Auslöseposition blockiert. Hierdurch wird vorteilhaft bewirkt, dass eine versehentliche Auslösung des Autoinjektors verhindert wird, solange die Abdeckkappe 30 an dem Gehäuse 10 befestigt ist.

Zur Vorbereitung der Verabreichung des Produkts wird die Abdeckkappe 30 von dem Gehäuse 10 entfernt. Durch das Entfernen der Abdeckkappe 30 wird gleichzeitig die Nadelschutzkappe 24, welche die Injektionsnadel 23 abdeckt, von dem Produktbehälter 20 entfernt. Im Ausgangs- oder Auslieferungszustand der Injektionsvorrichtung (Figur 1), d. h., wenn die Abdeckkappe 30 an der Injektionsvorrichtung angeordnet ist und noch nicht entfernt wurde, wird die Injektionsnadel 23 von der Nadelschutzkappe 24, die in dem gezeigten Beispiel als sogenanntes und dem Fachmann als solches bekanntes "rigid needle shield", alternativ als "soft needle shield", ausgebildet ist, abdeckt, um die Injektionsnadel 23 vor Verschmutzung zu schützen bzw. die Injektionsnadel 23 und das Medikament steril zu halten. Die Nadelschutzkappe 24 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter des Spritzenhalters ist zwischen dem Spritzenkörper und dem proximalen Ende der Nadelschutzkappe 24 angeordnet, insbesondere so, dass zwischen der Nadelschutzkappe 24 und der Schulter ein - wenngleich auch geringer - Spalt entsteht, um zu verhindern, dass die Schulter eine Kraft auf die Nadelschutzkappe 24 ausübt, wodurch zum Beispiel die Sterilität der Injektionsnadel 24 oder des flüssigen Produkts gefährdet werden könnte. Die Abdeckkappe 30 ist mit dem Gehäuse 10 oder einer Nadelschutzhülse 40 lösbar verschnappt, wobei diese Verschnappung gelöst wird, wenn die Abdeckkappe 30 von dem Gehäuse 10 oder dem Auslöseglied 40 entfernt wird. Die Verschnappung kann zum Beispiel durch eine Schnappgeometrie gebildet werden. Die Abdeckkappe 30 weist beispielsweise einen Schnapphaken auf, der einen Schnapper aufweist, der in eine Lücke zwischen dem Spritzenkörper, insbesondere dessen sich verjüngenden Bereich, und dem proximalen Ende der Nadelschutzkappe 24 eingreift. Wenn die Abdeckkappe 30 von dem Gehäuse 10 entfernt wird, hakt der Schnapper in das proximale Ende der Nadelschutzkappe 24 ein, wodurch die Nadelschutzkappe 24 von dem Produktbehälter 20 gelöst und zusammen mit der Abdeckkappe 30 von dem Gehäuse 10 entfernt wird. Alternativ können andere Mechanismen Anwendung finden, die die Abdeckkappe 30 mit der Nadelschutzkappe 24 derart verbinden, dass das Abnehmen der Abdeckkappe 30 vom Gehäuse 10 die Nadelschutzkappe 24 von dem Produktbehälter 20 entfernt.

In Figur 5 wird der vordere Teil der Injektionsvorrichtung dargestellt, wobei die Abdeckkappe 30 zusammen mit der Nadelschutzkappe 24 entfernt ist. Die Injektionsvorrichtung ist hierdurch für die Verabreichung vorbereitet. Für die Verabreichung wird das distale Ende des Auslöseglieds 40 an die gewünschte Einstichstelle des Patienten angesetzt. Das Gehäuse 10 wird vom Verwender zu der Einstichstelle hin gedrückt, wobei sich hierdurch das Auslöseglied 40 relativ zu dem Gehäuse 10 in die proximale Richtung bewegt. Dadurch, dass die Abdeckkappe 30 entfernt ist, wird das Sperrmittel 37 nicht mehr in die Sperrposition ausgelenkt. Das Sperrmittel 37 befindet sich in seiner Freigabeposition. Das Auslöseglied 40 kann somit bis in seine Auslöseposition verschoben werden, wobei die Kontaktfläche 37a an dem Anschlag 15 vorbei bewegt wird, da sich die Kontaktfläche 37a nicht in einer axialen Flucht entlang der Längsachse L mit dem Anschlag 15 befindet.

In der nicht gezeigten Auslöseposition steht die Injektionsnadel 23 über das distale Ende des Auslöseglieds 40 hervor, insbesondere mit einem Maß, welches der gewünschten Einstichtiefe entspricht. In der Ausgangsposition (Figur 5) wird die Injektionsnadel 23 von dem vorzugsweise hülsenförmigen Auslöseglied 40 umgeben. Insbesondere steht das Auslöseglied 40 distal über das distale Ende der Injektionsnadel 23, wenn sich das Auslöseglied 40 in seiner Ausgangsposition befindet.

Nach erfolgter Produktausschüttung kann eine zum Beispiel durch das Bewegen des Auslöseglieds 40 in die proximale Richtung gespannte Feder das Auslöseglied 40 relativ zu dem Gehäuse 10 in die distale Richtung verschieben, um eine Nadelschutzposition (nicht gezeigt) einzunehmen, in der das distale Ende des Auslöseglieds 40 über das distale Ende der Injektionsnadel 23 steht. In der Nadelschutzposition wird das Auslöseglied 40 vorzugsweise mit dem Gehäuse 10 so verrastet, dass es nicht mehr in die proximale Richtung verschiebbar ist, oder zumindest nicht mehr soweit verschiebbar ist, dass das distale Ende der Injektionsnadel 23 aus dem distalen Ende der Nadelschutzhülse 40 hervortritt.

Zusätzlich zu den Merkmalen der Vorrichtung aus der EP 2 745 866 A1 weist eine erfindungsgemäße Ausführung der Injektionsvorrichtung einen Mechanismus 12, 32 auf, der das Wiederanbringen der Abdeckkappe 30 an das distale Ende des Gehäuses 10 verhindert. Der Mechanismus 12, 32 oder die Injektionsvorrichtung weist einen Axialanschlag 12 und einen Axialgegenanschlag 32 auf. Der Axialanschlag 12 wird durch das Gehäuse 10, wie z. B. den hülsenförmigen Abschnitt 17 des Gehäuses 10 gebildet. Der Axialanschlag 12 kann beispielsweise von oder an dem distalen Ende des Gehäuses 10 gebildet werden. Der Axialgegenanschlag 32 ist von der Abdeckkappe 30, insbesondere einer Außenhülse 31, gebildet. Die Außenhülse 31 kann einteilig mit der Abdeckkappe 30 gebildet sein. In dem gezeigten Beispiel ist die Außenhülse 31 jedoch formschlüssig fest, wie z. B. ein durch eine Schnappverbindung mit einem Abdeckkappengrundkörper 35 verbundenes Element. Der Abdeckkappengrundkörper 35 kann beispielsweise an dem Gehäuse 10 befestigt sein, insbesondere mit dem Gehäuse 10 verschnappt oder anderweitig formschlüssig und/oder kraftschlüssig verbunden sein. Beispielsweise kann der Abdeckkappengrundkörper 35 ein Eingriffselement und das Gehäuse 10 ein Eingriffsgegenelement aufweisen, welche ineinandergreifen, wenn die Abdeckkappe 30, insbesondere der Abdeckkappengrundkörper 35 an dem Gehäuse 10 angeordnet oder befestigt sind.

Die Abdeckkappe 30, insbesondere die Außenhülse 31 weist einen elastisch nachgiebigen, d. h. federnden Arm 34 auf, der die Außenhülse 31 mit dem Axialgegenanschlag 32 verbindet.

Der Axialanschlag 32 ist aufgrund des elastisch nachgiebigen Arms 34 in etwa radial zur Längsachse L federnd angeordnet.

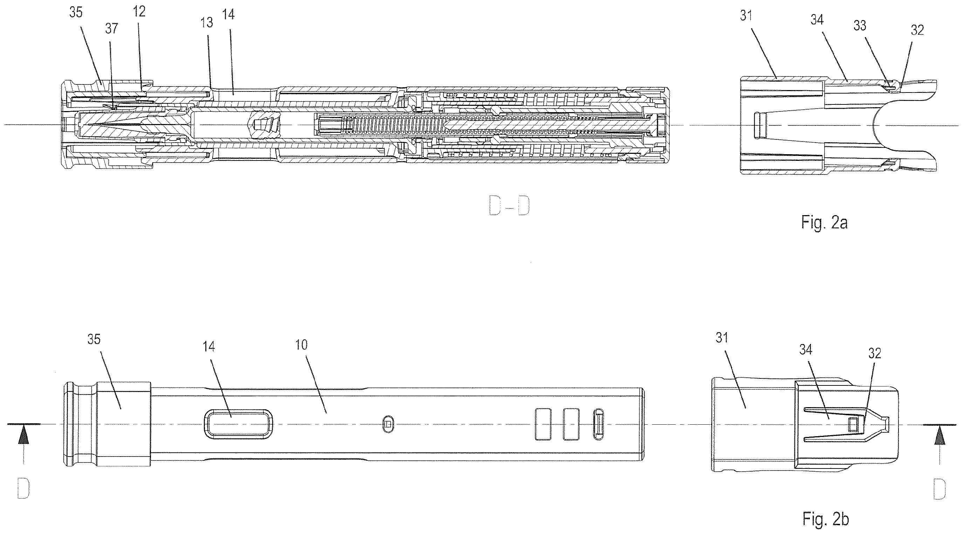

Wie aus den Figuren 2a und 2b erkennbar ist, kann die Außenhülse 31 über das proximale Ende der Injektionsvorrichtung, die als solche aus der EP 24 745 866 A1 bekannt ist, bis zu der Abdeckkappe 30 bzw. dem Abdeckkappengrundkörper 35 geschoben werden, bis die Außenhülse 31 mit dem Abdeckkappengrundkörper 35 axialfest, d. h. entlang der Längsachse L unverschiebbar mit dem Abdeckkappengrundkörper 35 verrastet. Alternativ könnte die Außenhülse 31 mit dem Abdeckkappengrundkörper 35 kraftschlüssig, wie z. B. reibschlüssig, oder stoffschlüssig, wie z. B. durch Verschweißen oder Kleben mit dem Abdeckkappengrundkörper verbunden werden. Alternativ kann die Außenhülse 31 über das distale Ende der Injektionsvorrichtung verschoben werden, bis es mit dem Abdeckkappengrundkörper 35 verbunden wird, insbesondere verschnappt. Durch das Aufschieben der Außenhülse 31 kann eine an sich bekannte Vorrichtung mit dem Mechanismus, der das Wiederanbringen der Abdeckkappe 30 an das distale Ende des Gehäuses 10 verhindert, nachgerüstet werden.

Die Figuren 1a und 1b zeigen die Außenhülse 31 in einem an dem Abdeckkappengrundkörper 35 befestigten Zustand, wobei der Abdeckkappengrundkörper 35 vollständig an dem Gehäuse 10 befestigt ist. In diesem Ausgangzustand oder montierten Zustand der Injektionsvorrichtung greift der Axialgegenanschlag in eine Ausnehmung 14 ein, die sich von dem Außenumfang des Gehäuses 10 nach innen erstreckt. In dem gezeigten Beispiel ist die Ausnehmung 14 gleichzeitig ein Sichtfenster, durch welches der Produktbehälter 20 inspiziert werden kann. Der Produktbehälterhalter 45 kann hierfür ebenfalls eine Ausnehmung aufweisen oder wie in dem gezeigten Beispiel aus einem transparenten Material, wie z. B. Kunststoff hergestellt sein, der eine Inspektion des Produktbehälters 20 durch die fensterförmige Ausnehmung 14 ermöglicht. Das distale Ende der Ausnehmung 14 weist eine Getriebefläche 13 auf, wobei unter Getriebefläche auch eine Getriebekannte verstanden wird, an der eine am Arm 34 nach innen ragende Abragung 33, welche den Axialgegenanschlag 32 bildet, abgleiten kann.

In dem montierten oder Ausgangszustand befindet sich der Axialgegenanschlag 32 proximal des Axialanschlags 12, d. h. wenn die Abdeckkappe 30 an dem Gehäuse 10 angeordnet ist. Aufgrund dessen, dass sich die Abragung 33 in der Ausnehmung 14 befindet, ist der Arm 34 entspannt.

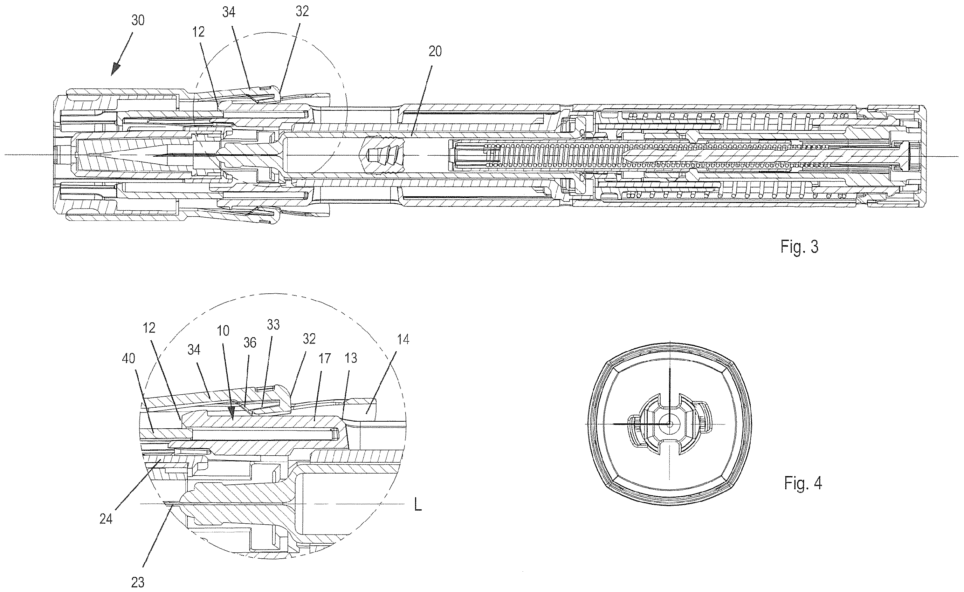

Wenn die Abdeckkappe 30 von dem Gehäuse 10 entfernt wird, wird der Axialanschlag 32 relativ zu dem Gehäuse 10 in die distale Richtung bewegt, wodurch die Abragung 33 oder eine nach distal weisende Getriebefläche oder Getriebegegenfläche 36 an der Getriebefläche 13 abgleitet, wodurch der Axialgegenanschlag, insbesondere die Abragung 33, in etwa radial nach außen, d. h. von der Längsachse L weg bewegt wird. Hierbei wird der elastisch nachgiebige Arm 34 gespannt (Figur 3). Die Abragung 33 gleitet nun an der Außenfläche des Gehäuses 10, insbesondere des Abschnitts 17 entlang. Der Abschnitt 17 kann z. B. eine oder mehrere Stufen aufweisen, welche von der Abragung 33 überfahren werden, insbesondere dadurch, dass die Getriebefläche oder Getriebegegenfläche 36 an der mindestens einen Stufe abgleitet. Wie weiter oben beschrieben, wird durch das Abnehmen der Abdeckkappe 30 vom Gehäuse 10 gleichzeitig die Nadelschutzkappe 24 von dem Nadelhalteabschnitt des Produktbehälters 20 entfernt. Wenn die Abdeckkappe 30 von dem Gehäuse 10 entfernt ist (Figur 5), wurde der Axialgegenanschlag 32 von dem Arm 34 federnd zur Längsachse L hinbewegt, wodurch er in einer axialen Flucht mit dem Axialanschlag 12 ist.

Bei dem Versuch, die Abdeckkappe 30 wieder an das Gehäuse 10 anzubringen oder an dem Gehäuse 10 zu befestigen, stößt der Axialanschlag 32, der sich entlang der Längsachse L in der Flucht mit dem Axialanschlag 12 befindet, an dem Axialanschlag 12 an, wodurch ein Wiederanbringen der Abdeckkappe 30 an das Gehäuse 10 verhindert wird.

Dadurch wird vorteilhaft erreicht, dass einerseits eine Verformung des Sperrmittels 37 durch ein fehlerhaftes Wiederanbringen der Abdeckkappe 30 an dem Gehäuse 10 verhindert wird und andererseits verhindert wird, dass der Anschein erweckt wird, dass die Nadel 23 noch steril sei, obwohl die Nadelschutzkappe 24 bereits die Injektionsnadel 23 in Bezug auf die Umgebung freigegeben hat.

### Bezugszeichenliste

- 10: Gehäuse
- 11: Vortriebsglied / Kolbenstange
- 12: Axialanschlag
- 13: Getriebefläche
- 14: Ausnehmung
- 15: Anschlag
- 16: zweiter Spalt
- 17: hülsenförmiger Abschnitt

- 20: Produktbehälter
- 21: Kolben
- 22: Produkt / Medikament
- 23: Injektionsnadel
- 24: Nadelabdeckkappe / Nadelschutzhülse
- 25: Produktbehälterabschnitt / Spritzenkörper
- 26: Fingerflansch

- 30: Abdeckkappe
- 31: Element / Außenhülse
- 32: Axialgegenanschlag
- 33: Abragung
- 34: Arm
- 35: Abdeckkappengrundkörper
- 36: Getriebefläche / Getriebegegenfläche
- 37: Sperrmittel
- 37a: Kontaktfläche
- 38: Eingriffselement
- 39: Innenhülse des Abdeckkappengrundkörpers
- 39a: Haltefläche

- 40: Auslöseglied
- 41: hülsenförmiger Abschnitt
- 42: erster Spalt
- 45: Produktbehälterhalter

- L: Längsachse

## Patentansprüche

1. Injektionsvorrichtung zur Ausschüttung eines flüssigen Produkts, insbesondere Medikaments, umfassend:
a) ein Gehäuse (10) mit einer Längsachse (L) und einen in dem Gehäuse (10) angeordneten Produktbehälter (20), insbesondere Spritze, der einen verschiebbaren Kolben (21) aufweist, wobei der Kolben (21) mittels eines Vortriebsglieds (11) zur Ausschüttung des in dem Produktbehälter (20) enthaltenen Produkts (22) entlang der Längsachse (L) in eine distale Richtung verschiebbar ist,
b) ein hülsenförmiges Auslöseglied (40), welches in seiner Ausgangsposition über das distale Ende des Gehäuses (10) ragt und für die Auslösung der Ausschüttung relativ zu dem Gehäuse (10) aus der Ausgangsposition in eine proximale Richtung in eine Auslöseposition bewegbar ist, wenn eine Abdeckkappe (30) von dem Gehäuse (10) abgenommen ist, und gegen die Bewegung in die Auslöseposition blockiert ist, wenn die Abdeckkappe (30) am Gehäuse (10) angeordnet ist,
**gekennzeichnet durch**
c) einen Axialanschlag (12), der von dem Gehäuse (10) oder einem sich an dem Gehäuse zumindest in die proximale Richtung abstützenden Element gebildet ist,
d) einen Axialgegenanschlag (32), der von der Abdeckkappe (30) oder einem sich an der Abdeckkappe (30) zumindest in die distale Richtung abstützenden Element (31) gebildet ist, wobei wenigstens einer aus Axialanschlag (12) und Axialgegenanschlag (32) durch das Abnehmen der Abdeckkappe (30) in eine Position gebracht wird, in der das Wiederanbringen der Abdeckkappe (30) an das distale Ende des Gehäuses (10) dadurch verhindert wird, dass der Axialanschlag (12) und Axialgegenanschlag (32) aneinander anstoßen.

2. Injektionsvorrichtung nach Anspruch 1, **dadurch gekennzeichnet, dass** der Produktbehälter (20) eine insbesondere unlösbar mit ihm verbundene Injektionsnadel (23) aufweist, wobei die Injektionsnadel (23) von einer Nadelschutzkappe (24) umgeben ist, die an dem Produktbehälter (20) lösbar befestigt ist.

3. Injektionsvorrichtung nach Anspruch 2, **dadurch gekennzeichnet, dass** die Abdeckkappe (30) mit der Nadelschutzkappe (24) derart verbunden ist, dass das Abnehmen der Abdeckkappe (30) die Nadelabdeckkappe (24) von dem Produktbehälter (20) entfernt.

4. Injektionsvorrichtung nach einem der vorhergehenden Ansprüche, **dadurch gekennzeichnet, dass** der Axialanschlag (12) federnd angeordnet ist und beim Abnehmen aus einer ausgelenkten Position in eine Sperrposition federt, wobei der Axialanschlag (12) in der Sperrposition das Wiederanbringen der Abdeckkappe (30) an das distale Ende des Gehäuses (10) dadurch verhindert, dass der Axialanschlag (12) und Axialgegenanschlag (32) aneinander anstoßen.

5. Injektionsvorrichtung nach einem der vorhergehenden Ansprüche, **dadurch gekennzeichnet, dass** der Axialgegenanschlag (32) federnd angeordnet ist und beim Abnehmen aus einer ausgelenkten Position in eine Sperrposition federt, wobei der Axialgegenanschlag (32) in der Sperrposition das Wiederanbringen der Abdeckkappe (30) an das distale Ende des Gehäuses (10) dadurch verhindert, dass der Axialanschlag (12) und Axialgegenanschlag (32) aneinander anstoßen.

6. Injektionsvorrichtung nach Anspruch 4 oder 5, **dadurch gekennzeichnet, dass** die Anordnung des Axialanschlags (12) oder des Axialgegenanschlags (32) so ausgestaltet ist, dass er aus der ausgelenkten Position quer, insbesondere im Wesentlichen radial zur Längsachse (L), oder in Umfangsrichtung um die Längsachse (L) in die Sperrposition federt.

7. Injektionsvorrichtung nach einem der Ansprüche 4 bis 6, **dadurch gekennzeichnet, dass** das Gehäuse (10) eine Getriebefläche (13) aufweist, welche den Axialgegenanschlag (32) während des Abnehmens der Abdeckkappe (30) von dem Gehäuse (10) aus einer Ausgangsposition in seine ausgelenkte Position federnd auslenkt.

8. Injektionsvorrichtung nach Anspruch 7, **dadurch gekennzeichnet, dass** das Gehäuse (10) an seinem Außenumfang eine Ausnehmung (14) aufweist, in die eine Abragung (33), die den Axialgegenanschlag (32) bildet, in ihrer Ausgangsposition eingreift, wobei das distale Ende der Ausnehmung (14) die Getriebefläche (13) bildet.

9. Injektionsvorrichtung nach einem der vorhergehenden Anspruch, **dadurch gekennzeichnet, dass** die Abdeckkappe (30) einen Abdeckkappengrundkörper (35) und eine Außenhülse (31), die mit dem Abdeckkappengrundkörper (35) dreh- und axialfest verbunden, insbesondere verschnappt ist, und den Axialgegenanschlag (32) bildet, aufweist.

10. Injektionsvorrichtung nach einem der vorhergehenden Ansprüche, **dadurch gekennzeichnet, dass** der Axialgegenanschlag (32) proximal des Axialanschlags (12) angeordnet ist, wenn die Abdeckkappe (30) an dem Gehäuse (10) angeordnet ist, und durch Abnehmen der Abdeckkappe (30) an dem Axialanschlag (12) entlang der Längsachse (L) vorbeibewegt wird.

11. Injektionsvorrichtung nach einem der vorhergehenden Ansprüche, **gekennzeichnet durch** eine Drehhülse, welche, insbesondere an dem Gehäuse (10), um die Längsachse (L) der Injektionsvorrichtung drehbar gelagert ist und an welcher der Axialanschlag (12) gebildet ist, wobei die Abdeckkappe (30) oder ein Teil der Abdeckkappe (30) während des Abnehmens der Abdeckkappe (30) an der Drehhülse abgleitet und sie um die Längsachse (L) dreht, wodurch der Axialanschlag (12) um die Längsachse (L) in eine Sperrposition gedreht wird, wobei der Axialanschlag (12) in der Sperrposition das Wiederanbringen der Abdeckkappe (30) an das distale Ende des Gehäuses (10) dadurch verhindert, dass der Axialanschlag (12) und Axialgegenanschlag (32) aneinander anstoßen.

12. Injektionsvorrichtung nach Anspruch 11, **dadurch gekennzeichnet, dass** das Auslöseglied (40) ein vorzugsweise federnd angeordnetes Sperrmittel (37) aufweist, wobei das Sperrmittel (37) von der Abdeckkappe (30) in eine Sperrposition, in der das Sperrmittel (37) einem von der Injektionsvorrichtung gebildeten Anschlag (12) gegenüberliegt, auslenkbar ist, wobei das Sperrmittel (37) in einer Freigabeposition, die das Sperrmittel (37) einnimmt, wenn die Abdeckkappe (30) abgenommen ist, an dem Anschlag (12) vorbeibewegbar ist.

## Claims

1. An injection device for discharging a fluid product, in particular a drug, including:
a) a housing (10) having a longitudinal axis (L) and a product container (20) arranged in the housing (10, in particular a syringe, which has a displaceable plunger (21), wherein the plunger (21) is displaceable along the longitudinal axis (L) in a distal direction by means of a forward drive member (11) for discharge of the product (22) contained in the product container (20),
b) a sleeve-shaped triggering member (40) which in its starting position projects beyond the distal end of the housing (10) and for triggering of the discharge is moveable relative to the housing (10) from the starting position in a proximal direction into a triggering position when a cover cap (30) is removed from the housing (10) and is blocked against movement into the triggering position when the cover cap (30) is arranged on the housing (10),
**characterised by**
c) an axial abutment (12) which is formed by the housing (10) or an element which is supported on the housing at least in the proximal direction, and
d) a counterpart axial abutment (32) which is formed by the cover cap (30) or an element (31) which is supported at the cover cap (30) at least in the distal direction, wherein at least one of the axial abutment (12) and the counterpart axial abutment (32) is moved by removal of the cover cap (30) into a position in which re-fitting of the cover cap (30) to the distal end of the housing (10) is prevented by the axial abutment (12) and the counterpart axial abutment (32) bearing against each other.

2. An injection device according to claim 1 **characterised in that** the product container (20) has an injection needle (23) which in particular is non-releasably connected thereto, wherein the injection needle (23) is surrounded by a needle protection cap (24) releasably fixed to the product container (20).

3. An injection device according to claim 2 **characterised in that** the cover cap (30) is connected to the needle protection cap (24) in such a way that removal of the cover cap (30) removes the needle cover cap (24) from the product container (20).

4. An injection device according to one of the preceding claims **characterised in that** the axial abutment (12) is arranged resiliently and upon removal resiliently moves from a deflected position into a locking position, wherein the axial abutment (12) in the locking position prevents re-fitting of the cover cap (30) to the distal end of the housing (10) by the axial abutment (12) and the counterpart axial abutment (32) bearing against each other.

5. An injection device according to one of the preceding claims **characterised in that** the counterpart axial abutment (32) is arranged resiliently and upon removal resiliently moves from a deflected position into a locking position, wherein the counterpart axial abutment (32) in the locking position prevents re-fitting of the cover cap (30) to the distal end of the housing (10) by the axial abutment (12) and the counterpart axial abutment (32) bearing against each other.

6. An injection device according to claim 4 or claim 5 **characterised in that** the arrangement of the axial abutment (12) or the counterpart axial abutment (32) is of such a configuration that it moves resiliently from the deflected position transversely, in particular substantially radially with respect to the longitudinal axis (L) or in a peripheral direction around the longitudinal axis (L), into the locking position.

7. An injection device according to one of claims 4 to 6 **characterised in that** the housing (10) has a drive surface (13) which resiliently deflects the counterpart axial abutment (32) from a starting position into its deflected position during removal of the cover cap (30) from the housing (10).

8. An injection device according to claim 7 **characterised in that** at its outer periphery the housing (10) has a recess (14) into which a projection (33) which forms the counterpart axial abutment (32) engages in its starting position, the distal end of the recess (14) forming the drive surface (13).

9. An injection device according to one of the preceding claims **characterised in that** the cover cap (30) has a cover cap base body (35) and an outer sleeve (31) which is non-rotatably and axially fixedly connected, in particular by a snap action, to the cover cap base body (35), and forms the counterpart axial abutment (32).

10. An injection device according to one of the preceding claims **characterised in that** the counterpart axial abutment (32) is arranged proximally of the axial abutment (12) when the cover cap (30) is arranged on the housing (10) and is advanced along the longitudinal axis (L) to the axial abutment (12) by removal of the cover cap (30).

11. An injection device according to one of the preceding claims **characterised by** a rotary sleeve which is mounted rotatably about the longitudinal axis (L) of the injection device in particular on the housing (10) and on which the axial abutment (12) is formed, wherein the cover cap (30) or a part of the cover cap (30) slides on the rotary sleeve during removal of the cover cap (30) and rotates same about the longitudinal axis (L), whereby the axial abutment (12) is rotated about the longitudinal axis (L) into a locking position, wherein the axial abutment (12) in the locking position prevents re-fitting of the cover cap (30) to the distal end of the housing (10) by the axial abutment (12) and the counterpart axial abutment (32) bearing against each other.

12. An injection device according to claim 11 **characterised in that** the triggering member (40) has a preferably resiliently arranged locking means (37), wherein the locking means (37) can be deflected by the cover cap (30) into a locking position in which the locking means (37) is opposite an abutment (12) formed by the injection device, wherein the locking means (37) is moveable past the abutment (12) in a release position which the locking means (37) assumes when the cover cap (30) is removed.

## Revendications

1. Dispositif d'injection pour la distribution d'un produit liquide, en particulier d'un médicament, comprenant :
a) un boîtier (10) avec un axe longitudinal (L) et un récipient de produit (20) agencé dans le boîtier (10), en particulier seringue, qui présente un piston coulissant (21), dans lequel le piston (21) peut coulisser au moyen d'un organe de propulsion (11) pour la distribution du produit (22) contenu dans le récipient de produit (20) le long de l'axe longitudinal (L) dans une direction distale,
b) un organe de déclenchement (40) en forme de douille, lequel dépasse dans sa position de départ de l'extrémité distale du boîtier (10) et est déplaçable pour le déclenchement de la distribution par rapport au boîtier (10) de la position de départ dans une direction proximale à une position de déclenchement, lorsqu'un capuchon protecteur (30) est retiré du boîtier (10), et est bloqué contre le déplacement dans la position de déclenchement, lorsque le capuchon protecteur (30) est agencé au niveau du boîtier (10),
**caractérisé par**
c) une butée axiale (12), qui est formée par le boîtier (10) ou un élément s'appuyant au moins dans la direction proximale contre le boîtier,
d) une contrebutée axiale (32), qui est formée par le capuchon protecteur (30) ou un élément (31) s'appuyant au moins dans la direction distale contre le capuchon protecteur (30), dans lequel au moins une parmi la butée axiale (12) et la contrebutée axiale (32) est amenée par le retrait du capuchon protecteur (30) dans une position, dans laquelle le remontage du capuchon protecteur (30) au niveau de l'extrémité distale du boîtier (10) est empêché par le fait que la butée axiale (12) et la contrebutée axiale (32) s'aboutent l'une à l'autre.

2. Dispositif d'injection selon la revendication 1, **caractérisé en ce que** le récipient de produit (20) présente une aiguille d'injection (23) reliée à celui-ci en particulier de manière inamovible, dans lequel l'aiguille d'injection (23) est entourée par un capuchon de protection d'aiguille (24), qui est fixé de manière amovible au récipient de produit (20).

3. Dispositif d'injection selon la revendication 2, **caractérisé en ce que** le capuchon protecteur (30) est relié au capuchon de protection d'aiguille (24) de sorte que le retrait du capuchon protecteur (30) enlève le capuchon de protection d'aiguille (24) du récipient de produit (20).

4. Dispositif d'injection selon l'une quelconque des revendications précédentes, **caractérisé en ce que** la butée axiale (12) est agencée de manière élastique et fait ressort lors du retrait d'une position déviée à une position de blocage, dans lequel la butée axiale (12) dans la position de blocage empêche le remontage du capuchon protecteur (30) au niveau de l'extrémité distale du boîtier (10) par le fait que la butée axiale (12) et la contrebutée axiale (32) s'aboutent l'une à l'autre.

5. Dispositif d'injection selon l'une quelconque des revendications précédentes, **caractérisé en ce que** la contrebutée axiale (32) est agencée de manière élastique et fait ressort lors du retrait d'une position déviée à une position de blocage, dans lequel la contrebutée axiale (32) dans la position de blocage empêche le remontage du capuchon protecteur (30) au niveau de l'extrémité distale du boîtier (10) par le fait que la butée axiale (12) et la contrebutée axiale (32) s'aboutent l'une à l'autre.

6. Dispositif d'injection selon la revendication 4 ou 5, **caractérisé en ce que** l'agencement de la butée axiale (12) ou de la contrebutée axiale (32) est configuré de sorte qu'elle fait ressort de la position déviée, transversalement, en particulier sensiblement radialement à l'axe longitudinal (L), ou dans la direction circonférentielle autour de l'axe longitudinal (L) dans la position de blocage.

7. Dispositif d'injection selon l'une quelconque des revendications 4 à 6, **caractérisé en ce que** le boîtier (10) présente une surface de transmission (13), laquelle dévie de manière élastique la contrebutée axiale (32) pendant le retrait du capuchon protecteur (30) du boîtier (10) d'une position de départ à sa position déviée.

8. Dispositif d'injection selon la revendication 7, **caractérisé en ce que** le boîtier (10) présente au niveau de sa circonférence extérieure un évidement (14), dans lequel une saillie (33), qui forme la contrebutée axiale (32), vient en prise dans sa position de départ, dans lequel l'extrémité distale de l'évidement (14) forme la surface de transmission (13).

9. Dispositif d'injection selon l'une quelconque des revendications précédentes, **caractérisé en ce que** le capuchon protecteur (30) présente un corps de base de capuchon protecteur (35) et une douille extérieure (31), qui est reliée solidaire en rotation et axialement solidaire au corps de base de capuchon protecteur (35), en particulier est enclenchée, et forme la contrebutée axiale (32).

10. Dispositif d'injection selon l'une quelconque des revendications précédentes, **caractérisé en ce que** la contrebutée axiale (32) est agencée de manière proximale à la butée axiale (12), lorsque le capuchon protecteur (30) est agencé au niveau du boîtier (10), et est déplacée par retrait du capuchon protecteur (30) devant la butée axiale (12) le long de l'axe longitudinal (L).

11. Dispositif d'injection selon l'une quelconque des revendications précédentes, **caractérisé par** une douille rotative, laquelle est logée en rotation autour de l'axe longitudinal (L) du dispositif d'injection, en particulier au niveau du boîtier (10), et au niveau de laquelle la butée axiale (12) est formée, dans lequel le capuchon protecteur (30) ou une partie du capuchon protecteur (30) glisse pendant le retrait du capuchon protecteur (30) au niveau de la douille rotative et elle tourne autour de l'axe longitudinal (L), ce par quoi la butée axiale (12) est tournée autour de l'axe longitudinal (L) dans une position de blocage, dans lequel la butée axiale (12) dans la position de blocage empêche le remontage du capuchon protecteur (30) au niveau de l'extrémité distale du boîtier (10) par le fait que la butée axiale (12) et la contrebutée axiale (32) s'aboutent l'une à l'autre.

12. Dispositif d'injection selon la revendication 11, **caractérisé en ce que** l'organe de déclenchement (40) présente un moyen de blocage (37) agencé de préférence de manière élastique, dans lequel le moyen de blocage (37) peut être dévié par le capuchon protecteur (30) dans une position de blocage, dans laquelle le moyen de blocage (37) fait face à une butée (12) formée par le dispositif d'injection, dans lequel le moyen de blocage (37) peut, dans une position de libération que le moyen de blocage (37) adopte, lorsque le capuchon protecteur (30) est retiré, être déplacé devant la butée (12).
